# EUROPÄISCHE PATENTSCHRIFT

(11) **EP 1 051 402 B1**
(45) Veröffentlichungstag und Bekanntmachung des Hinweises auf die Patenterteilung: **08.12.2004**
(21) Anmeldenummer: 99906156.7
(22) Anmeldetag: 19.01.1999
(51) Int. Cl.: C07D 233/90

(54) **VERFAHREN ZUR HERSTELLUNG VON S-ALKYL(ARYL)-SUBSTITUIERTEN IMIDAZOL-DERIVATEN**
METHOD FOR PRODUCING S-ALKYL(ARYL)-SUBSTITUTED IMIDAZOLE DERIVATIVES
PROCEDE DE FABRICATION DE DERIVES D'IMIDAZOLE S-ALKYL(ARYL) SUBSTITUE

(30) Priorität: 27.01.1998 DE 19802969
(43) Veröffentlichungstag der Anmeldung: 15.11.2000
(73) Patentinhaber: Aventis Pharma Deutschland GmbH, 65929 Frankfurt am Main (DE)
(72) Erfinder: HOLLA, Wolfgang, D-65779 Kelkheim (DE); NAPIERSKI, Bernd, D-65795 Hattersheim (DE); REBENSTOCK, Heinz-Peter, D-65439 Flörsheim (DE)
(86) Internationale Anmeldenummer: PCT/EP1999/000289
(87) Internationale Veröffentlichungsnummer: WO 1999/037620

(56) Entgegenhaltungen:
- EP-A- 0 014 834
- US-A- 5 482 957

## Beschreibung

Die Erfindung betrifft ein Verfahren zur Herstellung von S-Alkyl(Aryl)-substituierten Imidazol-Derivaten.

Bei der Herstellung von Wirkstoffen wie beispielsweise Arzneimittel für Herz- und Kreislauf-Erkrankungen haben sich S-Alkyl(Aryl)-substituierten Imidazol-Derivate als wichtige Zwischenprodukte im Herstellverfahren ergeben. Beispielsweise werden in US 5350751, US 5482957 oder US 5604251 die Herstellung von blutdrucksenkenden Präparaten beschrieben, welche als Wirkstoff Verbindungen vom Angiotensin II-Rezeptorenantagonist-Typ enthalten, die einen S-Alkyl(Aryl)-substituierten Imidazolrest aufweisen.

In der Europäischen Patentanmeldung Anmeldenummer 98100595.2 werden S-Alkyl(Aryl)-substituierten Imidazolderivate mit einer Biphenylsulfonylcyanamid-Seitenkette als natriumabhängige Chlorid/Bicarbonat-Austauscher vorgeschlagen, die infolge ihrer pharmakologischen Eigenschaften hervorragend als antiarrhythmische Arzneimittel mit kardioprotektiver Komponente u. a. zur Infarktprophylaxe, zur Infarktbehandlung sowie zur Behandlung von Angina pectoris geeignet sind.

Ein Verfahren zur Herstellung von S-Alkyl(Aryl)-substituierten Imidazolderivaten durch Umsetzung der azyklischen Aminoacrylsäureester-Vorstufe mit 4-Dimethylaminopyridin (DMAP) und PCl₅ zur entsprechenden Imidazolverbindung ist aus US 5350751, US 5482957. US 5604251 oder J. C. Caille et al.. Synthesis 1995. 635-637; P. Deprez et al.. J. Med. Chem. 1995, 38. 2357-2377 bekannt. Die Reinigung der erhaltenen S-Alkyl(Aryl)-Imidazolderivate erfolgt dann durch Chromatographie an Kieselgel. wobei die beschriebenen Ausbeuten bei 43-84 % liegen (J. C. Caille et al. supra).

Das bekannte Verfahren zur Zyklisierung und Reinigung der S-Alkyl(Aryl)-Imidazolderivate weist mehrere Nachteile auf.

So sind die erzielten Ausbeuten in vielen Fällen sehr niedrig. Ferner muß das zur Zyklisierung verwendete sehr teure Reagenz DMAP im großen Überschuß (ca. 2 Äquivalente) eingesetzt werden. Auch sind die S-Alkyl(Aryl)-Imidazolderivate nicht ohne aufwendige Chromatographie an Kieselgel in einer chemischen Reinheit > 99 % zu erhalten, Kristallisation bzw. Umkristallisation führt in der Mehrzahl der Fälle nicht zur gewünschten chemischen Reinheit; darüber hinaus sind dafür große Lösungsmittelmengen erforderlich. Das Verfahren ist daher zur Wirkstoffproduktion im technischen Maßstab (Herstellung von Kilo bis Tonnenmengen) wenig geeignet.

Aufgabe der vorliegenden Erfindung war die Entwicklung einer einfachen und ökonomischen Methode zur Zyklisierung der gut zugänglichen Aminoacrylsäureester (J. C. Caille et al., supra) zu den gewünschten S-Alkyl(Aryl)-Imidazolderivaten sowie einer praktikablen Methode zur Reinigung dieser Verbindungen.

Überraschenderweise wurde gefunden, daß sich S-Alkyl(Aryl)-Imidazolderivate durch Zyklisierung der entsprechenden Aminoacrylsäureester in Gegenwart von preiswerten und in technischen Mengen gut verfügbaren Alkylphosphonsäurenanhydriden. insbesondere von n-Propylphosphonsäureanhydrid (PPA) und anschließende Reinigung über ein geeignetes Salz in hohen Ausbeuten und sehr hohen Reinheiten herstellen lassen.

Die Erfindung betrifft daher ein Verfahren zur Herstellung von Verbindungen der allgemeinen Formel I, 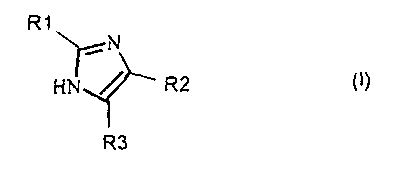 in welcher
- R(1): Wasserstoff;
Alkyl mit 1, 2, 3, 4, 5, 6, 7 oder 8 C-Atomen, das unsubstituiert ist oder substituiert ist mit 1, 2 oder 3 gleichen oder verschiedenen Resten aus der Reihe F, Cl, Br, CF₃, Methyl, Methoxy, NO₂ oder NR(6)R(7);
- CₙH₂ₙ-Cycloalkyl mit 1, 2, 3, 4, 5, 6 oder 7-C-Atomen;
mit n gleich Null, 1, 2 oder 3;
-CₙH₂ₙ -Phenyl, das unsubstituiert ist oder substituiert ist mit 1, 2 oder 3 gleichen oder verschiedenen Resten aus der Reihe F, Cl, Br, CF₃, NO₂, Methyl, Methoxy, Hydroxy oder NR(6)R(7); mit n gleich Null, 1, 2 oder 3;
- CₙH₂ₙ-Heteroaryl mit 1, 2, 3, 4, 5, 6, 7, 8 oder 9 C-Atomen, das unsubstituiert ist oder substituiert ist mit 1, 2 oder 3 gleichen oder verschiedenen Resten aus der Reihe F, Cl, Br, CF₃, NO₂, Methyl, Methoxy, Hydroxy oder NR(6)R(7);
mit n gleich Null, 1, 2 oder 3;
- R(2) und R(3): unabhängig voneinander
- SR(4) oder -COOR(5);
- R(4) und R(5): unabhängig voneinander
Alkyl mit 1, 2, 3, 4, 5, 6, 7 oder 8 C-Atomen, das unsubstituiert ist oder substituiert ist mit 1, 2 oder 3 gleichen oder verschiedenen Resten aus der Reihe F. Cl, Br, CF₃, Methyl. NO₂, Methoxy oder NR(6)R(7);
- CₙH₂ₙ-Cycloalkyl mit 3, 4, 5, 6 oder 7 C-Atomen: mit n gleich Null, 1, 2 oder 3;
- CₙH₂ₙ-Phenyl, das unsubstituiert ist oder substituiert ist mit 1, 2 oder 3 gleichen oder verschiedenen Resten aus der Reihe F, Cl, Br, CF₃, CN, NO₂, Methyl, Methoxy, Hydroxy oder NR(6)R(7); mit n gleich Null, 1, 2 oder 3;
-CₙH₂ₙ-Heteroaryl mit 1, 2, 3, 4, 5, 6, 7, 8 oder 9 C-Atomen, das unsubstituiert ist oder substituiert ist mit 1, 2 oder 3 gleichen oder verschiedenen Resten aus der Reihe F, Cl, Br, CF₃, NO₂, Methyl, Methoxy, Hydroxy oder NR(6)R(7);
mit n gleich Null, 1, 2 oder 3;
- R(6) und R(7): unabhängig voneinander
Alkyl mit 1, 2, 3 oder 4 C-Atomen;
oder ein Salz davon bedeuten,
dadurch gekennzeichnet, daß man Verbindungen der Formel II 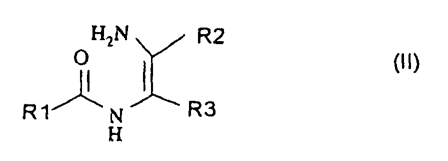 in welcher R(1 ), R(2) und R(3) die oben definierte Bedeutung haben, in Gegenwart von Alkylphosphonsäureanhydriden zu Verbindungen der Formel (I) zyklisiert, diese anschließend in an sich bekannterweise durch Salzbildung und
gegebenenfalls anschließender Umkristallisierung aufreinigt und gegebenenfalls zum Schutz anderer funktioneller Gruppen eingeführte Reste in an sich bekannter Weise abspaltet.

Das erfindungsgemäß verwendete Alkylphosphonsäureanhydrid ist bei Raumtemperatur stabil. In den meisten nicht wäßrigen Lösungsmittel, insbesondere in Lipidlösungsmitteln wie Chloroform oder Methylenchlorid, aber auch in polaren Lösungsmitteln wie DMF und DMA ist es leicht löslich.

Die Herstellung des Allkylphosphonsäureanhydrids kann in an sich bekannter Weise erfolgen, wie z.B. in Houben-Weyl, Methoden der Organischen Chemie, G. Thieme Verl., Stuttgart 1963, Bd. XII, S. 612 formuliert. Die Herstellung von n-Propylphosphansäureanhydrid (PPA) ist beispielsweise nach dem von Wissmann und Kleiner beschriebenen Verfahren möglich (Angew. Chem. 92 (1980) Nr. 2, S. 129-130).

Die für die Herstellung der Verbindungen der Formel (I) notwendigen azyklischen Vorstufen (II) sind nach literaturbekannten Methoden (J. C. Caille et al., Synthesis 1995, 635; P. Deprez et al., J. Med. Chem. 1995, 38, 2357) leicht herzustellen.

Eine bevorzugte Ausführungsform ist dadurch gekennzeichnet, daß Verbindungen der Formel I hergestellt werden, in der
- R(1): Wasserstoff;
Alkyl mit 1, 2, 3, 4, 5, 6, 7 oder 8 C-Atomen, das unsubstituiert ist oder substituiert ist mit 1 oder 2 gleichen oder verschiedenen Resten aus der Reihe F, Cl, CF₃, Methyl, Methoxy, NO₂ oder NR(6)R(7);
- CₙH₂ₙ-Cycloalkyl mit 1, 2, 3, 4, 5, 6 oder 7-C-Atomen;
mit n gleich Null, 1, oder 2:
-CₙH₂ₙ-Phenyl, das unsubstituiert ist oder substituiert ist mit 1 oder 2 gleichen oder verschiedenen Resten aus der Reihe F, Cl, CF₃, NO₂, Methyl, Methoxy, Hydroxy oder NR(6)R(7);
mit n gleich Null, 1 oder 2;
- CₙH₂ₙ-Heteroaryl mit 1, 2, 3, 4, 5, 6, 7, 8 oder 9 C-Atomen, das unsubstituiert ist oder substituiert ist mit einem Rest aus der Reihe F, Cl, CF₃, NO₂, Methyl, Methoxy, Hydroxy oder NR(6)R(7);
mit n gleich Null, 1 oder 2;
- R(2) und R(3): unabhängig voneinander
- SR(4) oder -COOR(5);
- R(4) und R(5): unabhängig voneinander
Alkyl mit 1, 2, 3, 4, 5, 6, 7 oder 8 C-Atomen, das unsubstituiert ist oder substituiert ist mit 1 oder 2 gleichen oder verschiedenen Resten aus der Reihe F, Cl, CF₃, Methyl, NO₂, Methoxy oder NR(6)R(7);
- CₙH₂ₙ-Cycloalkyl mit 3, 4, 5, 6 oder 7 C-Atomen;
mit n gleich Null, 1 oder 2;
- CₙH₂ₙ-Phenyl, das unsubstituiert ist oder substituiert ist mit 1 oder 2 gleichen oder verschiedenen Resten aus der Reihe F, Cl, CF₃, CN, NO₂, Methyl, Methoxy, Hydroxy oder NR(6)R(7); mit n gleich Null, 1 oder 2;
- CₙH₂ₙ-Heteroaryl mit 1, 2, 3, 4, 5, 6, 7, 8 oder 9 C-Atomen, das unsubstituiert ist oder substituiert ist mit einem Rest aus der Reihe F, Cl, CF₃, NO₂, Methyl, Methoxy, Hydroxy oder NR(6)R(7); mit n gleich Null. 1 oder 2:
- R(6) und R(7): Methyl;
oder ein Salz davon bedeuten.

Besonders bevorzugt ist eine Ausführungsform, die dadurch gekennzeichnet ist, daß Verbindungen der Formel I hergestellt werden, in der
- R(1): Wasserstoff;
Alkyl mit 1, 2, 3, 4, 5, 6, 7 oder 8 C-Atomen, das unsubstituiert ist oder substituiert ist mit 1 einem Rest aus der Reihe F, Cl, CF₃, Methyl, Methoxy, NO₂ oder NR(6)R(7);
Cycloalkyl mit 1, 2, 3, 4, 5, 6 oder 7-C-Atomen;
Phenyl, das unsubstituiert ist oder substituiert ist mit einem Rest aus der Reihe F, Cl, CF₃, NO₂, Methyl, Methoxy, Hydroxy oder NR(6)R(7);
Heteroaryl mit 1, 2, 3, 4, 5, 6, 7, 8 oder 9 C-Atomen, das
unsubstituiert ist oder substituiert ist mit einem Rest aus der Reihe F, Cl, CF₃, NO₂, Methyl, Methoxy, Hydroxy oder NR(6)R(7);
- R(2) und R(3): unabhängig voneinander
- SR(4) oder -COOR(5);
- R(4) und R(5): unabhängig voneinander
Alkyl mit 1, 2, 3, 4, 5, 6, 7 oder 8 C-Atomen, das unsubstituiert ist oder substituiert ist mit einem Rest aus der Reihe F, Cl, CF₃, Methyl, NO₂, Methoxy oder NR(6)R(7);
Cycloalkyl mit 3, 4, 5, 6 oder 7 C-Atomen;
Phenyl, das unsubstituiert ist oder substituiert ist mit einem Rest aus der Reihe F, Cl, CF₃, CN, NO₂, Methyl, Methoxy, Hydroxy oder NR(6)R(7);
Heteroaryl mit 1, 2, 3, 4, 5, 6, 7, 8 oder 9 C-Atomen, das
unsubstituiert ist oder substituiert ist mit einem Rest aus der Reihe F, Cl, CF₃, NO₂, Methyl, Methoxy, Hydroxy oder NR(6)R(7);
- R(6) und R(7): Methyl;
oder ein Salz davon bedeuten.

Ganz besonders bevorzugt ist eine Ausführungsform, die dadurch gekennzeichnet ist, daß Verbindungen der Formel I hergestellt werden, in der
- R(1): Wasserstoff;
Alkyl mit 1, 2, 3, 4, 5 oder 6 C-Atomen, das unsubstituiert ist oder substituiert ist mit 1 einem Rest aus der Reihe F, Cl, CF₃, Methyl oder Methoxy;
Cycloalkyl mit 1, 2, 3, 4, 5, 6 oder 7-C-Atomen;
Phenyl, das unsubstituiert ist oder substituiert ist mit einem Rest aus der Reihe F, Cl, CF₃, NO₂, Methyl oder Methoxy;
Heteroaryl mit 1, 2, 3, 4, 5, 6, 7, 8 oder 9 C-Atomen. das unsubstituiert ist oder substituiert ist mit einem Rest aus der Reihe F, Cl, CF₃, Methyl oder Methoxy;
- R(2) und R(3): unabhängig voneinander
- SR(4) oder -COOR(5);
- R(4) und R(5): unabhängig voneinander
Alkyl mit 1, 2, 3, 4, 5 oder 6 C-Atomen, das unsubstituiert ist oder substituiert ist mit einem Rest aus der Reihe F, Cl, CF₃, Methyl oder Methoxy;
Cycloalkyl mit 3, 4, 5, 6 oder 7 C-Atomen;
Phenyl, das unsubstituiert ist oder substituiert ist mit einem Rest aus der Reihe F, Cl, CF₃, CN, Methyl oder Methoxy;
Heteroaryl mit 1, 2, 3, 4, 5, 6, 7, 8 oder 9 C-Atomen, das unsubstituiert ist oder substituiert ist mit einem Rest aus der Reihe F. Cl. CF_{3,} Methyl oder Methoxy;
oder ein Salz davon bedeuten.

Bevorzugt ist auch die Herstellung von Verbindungen der Formel I, in denen R(1 ) Alkyl mit 1, 2, 3, 4, 5 oder 6 C-Atomen bedeutet und die anderen Reste und Variablen wie oben definiert sind, sowie deren Salze.

Bevorzugt ist desweiteren auch die Herstellung von Verbindungen der Formel I, in denen
- R(1): Alkyl mit 1, 2, 3, 4, 5 oder 6 C-Atomen bedeutet;
- R(2) und R(3): unabhängig voneinander
- SR(4) oder -COOR(5);
- R(4): Alkyl mit 1, 2, 3, 4, 5 oder 6 C-Atomen oder -CₙH₂ₙ-Phenyl mit n gleich Null, 1 oder 2 bedeutet und
- R(5): Alkyl mit 1, 2, 3, 4, 5 oder 6 C-Atomen
oder ein Salz davon, bedeutet.

Bevorzugt ist desweiteren die Herstellung von Verbindungen der Formel I oder ein Salz davon, in der R(2) -S(R4) und R(3) -COOR(5) bedeutet, wobei die anderen Reste und Variablen wie oben definiert sind.

Alkyl kann geradkettig oder verzweigt sein. Beipiele für Alkylreste mit 1, 2, 3, 4, 5, 6, 7 oder 8 C-Atomen sind: Methyl, Ethyl, Propyl, Butyl, Pentyl, Hexyl, Heptyl, Octyl, Isopropyl, Isobutyl, Isopentyl, Neopentyl, Isohexyl, 3-Methylpentyl, sec-Butyl, tert-Butyl, tert-Pentyl.

Cycloalkylreste sind insbesondere Cyclopropyl, Cyclobutyl, Cyclopentyl, Cyclohexyl, Cycloheptyl, die aber auch beispiesweise durch Alkyl mit 1, 2, 3 oder 4 C-Atomen substituiert sein können. Als Beispiel für substituierte Cycloalkylreste seien 4-Methylcyclohexyl und 2,3-Dimethylcyclopentyl genannt.

Unter Heteroaryl werden insbesondere Reste verstanden, die sich von Phenyl oder Naphthyl ableiten, in weichen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.
Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Gegebenenfalls vorkommende Stereozentren können sowohl (R)- als auch (S)-konfiguriert sein.
Die Verbindungen der allgemeinen Formel II können als E/Z-Isomerengemische oder als reine E- bzw. Z- Isomere eingesetzt werden. E/Z-Isomere können durch Chromatographie in die Einzelisomere aufgetrennt werden.

Die Zyklisierung der Verbindungen der Formel (II) zu Verbindungen der Formel (I) erfolgt vorzugsweise durch Umsetzung der azyklischen Verbindungen mit dem preiswerten und kommerziell erhältlichen n-Propylphosphonsäureanhydrid (PPA, H. Wissmann u. H.-J. Kleiner, Angew. Chem. 1980, 92, 129) in einem geeigneten Lösungsmittel. Man erhält die gewünschten Verbindungen der Formel (I) als Rohprodukte durch Neutralisation der Reaktionsmischung. Zur Isolierung der reinen Verbindungen der Formel (I) wird das Rohprodukt in einem geeigneten organischen Lösungsmittel unter leichter Kühlung mit einer geeigneten Säure wie beispielsweise Schwefelsäure versetzt. Das ausfallende Salz wird abgesaugt und gewaschen oder aus geeigneten Lösungsmitteln umkristallisiert. Nach Umsetzung des reinen Salzes mit Base erhält man die Verbindungen der Formel (I) in hoher Ausbeute und hoher chemischer Reinheit.

Die Zyklisierung kann in den verschiedensten Lösungsmittel durchgeführt werden. Geeignete Lösungsmittel sind beispielsweise Ester wie Essigsäureethylester, tert.-Butylacetat, Ether wie z. B. tert.-Butylmethylether. Dioxan, THF. Kohlenwasserstoffe und halogenierte Kohlenwasserstoffe wie Toluol und Methylenchlorid. Es können auch Gemische verschiedener Lösungsmittel verwendet werden, wie beispielsweise Toluol/Ethylacetat.

Die Reihenfolge der Reagenzien-Zugabe ist unerheblich. Es kann sowohl die azyklische Verbindungen der Formel (II) als auch das Alkylphosphonsäureanhydrid, z. B. PPA zuerst vorgelegt werden. Es ist auch möglich beide Komponenten gleichzeitig in das Reaktionsgefäß zu zudosieren. Vielfach ist es sinnvoll die kommerziell erhältlichen Lösungen von PPA, z. B. in Ethylacetat, zu verwenden.

Die verwendete Alkylphosphonsäureanhydrid-Menge liegt zwischen 0.1-5.0 Mol Alkylphosphonsäureanhydrid pro Mol Verbindungen der Formel (II), bevorzugt bei 0.2-1.0 Mol Alkylphosphonsäureanhydrid.

Die Zyklisierung wird im allgemeinen bei Temperaturen von 10-130 °C, bevorzugt bei 20-80 °C, durchgeführt.

Die Reaktionsdauer liegt zwischen 0.25 h und 3 Tagen, bevorzugt zwischen 1 und 48 h.

Zum Neutralisieren kommen sowohl anorganische als auch organische Basen in Betracht, beispielsweise Ammoniak oder Amine wie z. B. Triethylamin oder Salze wie z. B. K₂CO₃ oder NaHCO₃.

Die Verbindungen der Formel I können als Salze der Schwefelsäure, z.B. als Sulfate oder Hydrogensulfate vorliegen aber auch als Salze anderer, zum Ausfällen der Imidazolverbindungen verwendbarer Säuren.

Die Verbindungen der Formel I sind wichtige Zwischenprodukte für die Herstellung von pharmakologisch wirksamen Substanzen, wie beispielsweise Angiotensin II-Rezeptorantagonisten oder natriumabhängige Chlorid/Bicarbonat-Austauscher (NCBE). Die Überführung der Verbindungen der Formel zu pharmakologisch wirksamen Substanzen kann dabei beispielsweise gemäß den in US 5350751, US 5482957, US 5604251, der europäischen Patentanmeldung Nr. 98100595.2 oder P. Deprez et al., J. Med. Chem. 1995, 38, 2357-2377 beschriebenen Syntheseverfahren erfolgen.

### Beispiele:

### Beispiel 1:

### 2-n-Butyl-4-methylthio-1 H-imidazol-5-carbonsäureethylester

14 L 50 %ige PPA-Lösung (22.9 mol) in Ethylacetat werden in 100 L Ethylacetat vorgelegt und 30 min bei ca 20 °C gerührt. Danach werden 10.0 kg (38.42 mol) (*E*)-und (Z)-3-Amino-3-thiomethyl-2(n-butylcarbonyl)-aminoacrylsäureethylester eingetragen. Man rührt bei ca. 20 °C bis alles gelöst ist und erhitzt zum Rückfluß. Nach ca. 2 h ist die Reaktion beendet. Man kühlt auf 20 °C ab, neutralisiert das Reaktionsgemisch vorsichtig mit 60 L ges. NaHCO₃-Lsg. und trennt die Phasen. Anschließend wird die organische Phase auf 10 °C gekühlt und mit 2,05 L konz. Schwefelsäure versetzt. Man rührt bis zur vollständigen Kristallisation (ca. 20 min), saugt dann das Imidazol-Salz ab und trocknet es bei 20 - 30 °C im Vakuum; Ausbeute Imidazol-Salz: 11.9 kg (91 %); mp 150-160°C.

Zur Freisetzung des gewünschten Imidazols werden 11,9 kg 2-n-Butyl-4-methylthio-1 H-imidazol-5-carbonsäureethylester x H₂SO₄ in 65 L Wasser gelöst, anschließend mit 30 L MTB-ether und dann zur Neutralisation portionsweise mit 6.2 kg Natriumhydrogencarbonat versetzt. Die Phasen werden getrennt. Die organische Phase wird gründlich mit Na₂SO₄ getrocknet. Das Filtrat wird zur Trockne eingeengt. Der Rückstand wird mit 50 L Petrolether versetzt und zum Rückfluß erhitzt, bis alles gelöst ist. Man kühlt langsam auf -5 bis 0 °C ab, rührt eine weitere Stunde und filtriert dann das ausgefallene Produkt ab. Der erhaltene Feststoff wird mit wenig, gekühltem Petrolether nachgewaschen. Das Produkt wird bei 20 - 30 °C im Vakuum getrocknet. Ausbeute: 7.85 kg (92.6 %).

| | | | | |
|---|---|---|---|---|
| C₁₁H₁₈N₂O₂S ber. | C 54.51 | H 7.48 | N 11.56 | S 13.23 |
| gef. | C 54.5 | H 7.5 | N 11.6 | S 13.3 |

Mp: 74-76°C.
1H-NMR (200 MHz, CDCl3): δ = 0.93 (t, J = 7.5 Hz; 3 H), 1.4 (m; 5 H), 1.74 (m; 2 H), 2.6 (s; 3 H), 2.73 (t, J = 7.5 Hz: 2 H), 4.35 (q, J = 7 Hz; 2 H).

Die Verbindungen der Beispiele 2 bis 5 können wie folgt hergestellt werden.

### Beispiel 2:

### 2-Methyl-4-ethylthio-1 H-imidazol-5-carbonsäureethylester

14 ml 50 %ige PPA-Lösung (0,023 mmol) in Ethylacetat werden in 100 ml Ethylacetat vorgelegt und 30 min bei ca 20 °C gerührt. Danach werden 8.83 g (0,038 mol) *(E)-* und (2)-3-Amino-3-thioethyl-2(methylcarbonyl)-aminoacrylsäureethylester eingetragen. Man rührt bei ca. 20 °C bis alles gelöst ist und erhitzt zum Rückfluß. Nach ca. 2 h ist die Reaktion beendet. Man kühlt auf 20 °C ab, neutralisiert das Reaktionsgemisch vorsichtig mit 60 ml ges. NaHCO₃-Lsg. und trennt die Phasen. Anschließend wird die organische Phase auf 10 °C gekühlt und mit ca. 2 ml konz. Schwefelsäure versetzt. Man rührt bis zur vollständigen Kristallisation, saugt dann das Imidazol-Salz ab und trocknet es bei 20 - 30 °C im Vakuum.
Zur Freisetzung des gewünschten Imidazols werden 10,9 g (0,035 mol) 2-Methyl-4-ethylthio-1 H-imidazol-5-carbonsäureethylester x H₂SO₄ in ca. 70 ml Wasser gelöst, anschließend mit 30-40 ml MTB-ether und dann zur Neutralisation portionsweise mit ca. 6.2 g Natriumhydrogencarbonat versetzt. Die Phasen werden getrennt. Die organische Phase wird gründlich mit Na₂SO₄ getrocknet. Das Filtrat wird zur Trockne eingeengt. Der Rückstand wird mit 50-60 ml Petrolether versetzt und zum Rückfluß erhitzt, bis alles gelöst ist. Man kühlt langsam auf -5 bis 0 °C ab, rührt eine weitere Stunde und filtriert dann das ausgefallene Produkt ab. Der erhaltene Feststoff wird mit wenig, gekühltem Petrolether nachgewaschen. Das Produkt wird bei 20 - 30 °C im Vakuum getrocknet.

### Beispiel 3:

### 2-n-Propyl-4-ethylthio-1 H-imidazol-5-carbonsäureethylester

14 ml 50 %ige PPA-Lösung (0,023 mol) in Ethylacetat werden in 100 ml Ethylacetat vorgelegt und 30 min bei ca 20 °C gerührt. Danach werden 10 g (0,038 mol) *(E)-* und (Z)-3-Amino-3-thioethyl-2(n-Propylcarbonyl)-aminoacrylsäureethylester eingetragen. Man rührt bei ca. 20 °C bis alles gelöst ist und erhitzt zum Rückfluß. Nach ca. 2 h ist die Reaktion beendet. Man kühlt auf 20 °C ab, neutralisiert das Reaktionsgemisch mit 60 ml ges. NaHCO₃-Lsg. und trennt die Phasen. Anschließend wird die organische Phase auf 10 °C gekühlt und mit ca. 2 ml konz. Schwefelsäure versetzt. Man rührt bis zur vollständigen Kristallisation, saugt dann das Imidazol-Salz ab und trocknet es bei 20 - 30 °C im Vakuum.

Zur Freisetzung des gewünschten Imidazols werden 11,6 g 2-n-Propyl-4-ethylthio-1 H-imidazol-5-carbonsäureethylester x H₂SO₄ in ca. 70 ml Wasser gelöst, anschließend mit 30-40 ml MTB-ether und dann zur Neutralisation portionsweise mit ca. 6.2 g Natriumhydrogencarbonat versetzt. Die Phasen werden getrennt. Die organische Phase wird gründlich mit Na₂SO₄ getrocknet. Das Filtrat wird zur Trockne eingeengt. Der Rückstand wird mit 50 - 60 ml Petrolether versetzt und zum Rückfluß erhitzt, bis alles gelöst ist. Man kühlt langsam auf -5 bis 0 °C ab, rührt eine weitere Stunde und filtriert dann das ausgefallene Produkt ab. Der erhaltene Feststoff wird mit wenig, gekühltem Petrolether nachgewaschen. Das Produkt wird bei 20 - 30 °C im Vakuum getrocknet.

### Beispiel 4:

### 2-n-Butyl-4-ethylthio-1 H-imidazol-5-carbonsäuremethylester

Die Herstellung kann analog zu Beispiel 3 durchgefüht werden. Statt (*E*)- und (*Z*)-3-Amino-3-thioethyl-2(n-Propylcarbonyl)-aminoacrylsäureethylester werden dann 0,038 Mol (*E*)- und (*Z*)-3-Amino-3-thioethyl-2(n-Butylcarbonyl)-aminoacrylsäuremethylester eingetragen.

### Beispiel 5:

### 2-n-Butyl-4-benzylthio-1H-imidazol-5-carbonsäureethylester

Die Herstellung kann analog zu Beispiel 3 durchgeführt werden. Statt (*E*)- und (*Z*)-3-Amino-3-thioethyl-2(n-Propylcarbonyl)-aminoacrylsäureethylester werden dann 12,8 g (0,038 Mol) (*E*)- und (*Z*)-3-Amino-3-thiobenzyl-2(n-Butylcarbonyl)-aminoacrylsäureethylester eingetragen.

## Patentansprüche

1. Verfahren zur Herstellung von Verbindungen der allgemeinen Formel I, 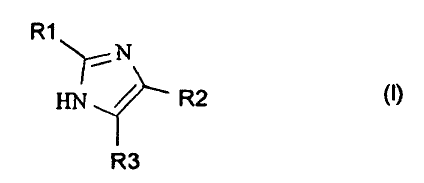 in welcher
R(1) Wasserstoff;
Alkyl mit 1, 2, 3, 4, 5, 6, 7 oder 8 C-Atomen, das unsubstituiert ist oder substituiert ist mit 1, 2 oder 3 gleichen oder verschiedenen Resten aus der Reihe F, Cl, Br, CF₃, Methyl, Methoxy, NO₂ oder NR(6)R(7);
-CₙH₂ₙ-Cycloalkyl mit 1, 2, 3, 4, 5, 6 oder 7-C-Atomen; mit n gleich Null, 1, 2 oder 3;
- CₙH₂ₙ -Phenyl, das unsubstituiert ist oder substituiert ist mit 1, 2 oder 3 gleichen oder verschiedenen Resten aus der Reihe F, Cl, Br, CF₃, NO₂, Methyl, Methoxy, Hydroxy oder NR(6)R(7);
mit n gleich Null, 1, 2 oder 3;
- CₙH₂ₙ-Heteroaryl mit 1, 2, 3, 4, 5, 6, 7, 8 oder 9 C-Atomen, das unsubstituiert ist oder substituiert ist mit 1, 2 oder 3 gleichen oder verschiedenen Resten aus der Reihe F, Cl, Br, CF₃, NO₂, Methyl, Methoxy, Hydroxy oder NR(6)R(7);
mit n gleich Null, 1, 2 oder 3;
R(2) und R(3) unabhängig voneinander
- SR(4) oder -COOR(5);
R(4) und R(5) unabhängig voneinander
Alkyl mit 1, 2, 3, 4, 5, 6, 7 oder 8 C-Atomen, das unsubstituiert ist oder substituiert ist mit 1, 2 oder 3 gleichen oder verschiedenen Resten aus der Reihe F, Cl, Br, CF₃, Methyl, NO₂, Methoxy oder NR(6)R(7);
- CₙH₂ₙ-Cycloalkyl mit 3, 4, 5, 6 oder 7 C-Atomen;
mit n gleich Null, 1, 2 oder 3;
- CₙH₂ₙ-Phenyl, das unsubstituiert ist oder substituiert ist mit 1, 2 oder 3 gleichen oder verschiedenen Resten aus der Reihe F, Cl, Br, CF₃, CN, NO₂, Methyl, Methoxy, Hydroxy oder NR(6)R(7);
mit n gleich Null, 1, 2 oder 3;
- CₙH₂ₙ-Heteroaryl mit 1, 2, 3, 4, 5, 6, 7, 8 oder 9 C-Atomen, das unsubstituiert ist oder substituiert ist mit 1, 2 oder 3 gleichen oder verschiedenen Resten aus der Reihe F, Cl, Br, CF₃, NO₂, Methyl, Methoxy, Hydroxy oder NR(6)R(7);
mit n gleich Null, 1, 2 oder 3;
R(6) und R(7) unabhängig voneinander Alkyl mit 1, 2, 3 oder 4 C-Atomen;
oder ein Salz davon bedeuten,
**dadurch gekennzeichnet, daß** man Verbindungen der Formel II 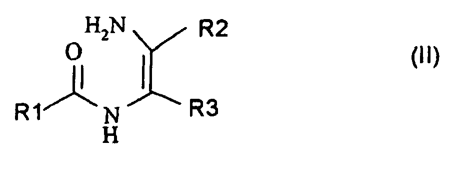 in welcher R(1 ), R(2) und R(3) die oben definierte Bedeutung haben, in Gegenwart von n-Propylphosphonsäureanhydrid zu Verbindungen der Formel (I) zyklisiert, diese anschließend durch Salzbildung und gegebenenfalls anschließender Umkristallisierung aufreinigt und gegebenenfalls zum Schutz anderer funktioneller Gruppen eingeführte Reste abspaltet.

2. Verfahren gemäß Anspruch 1, **dadurch gekennzeichnet, daß** Verbindungen der Formel I oder ein Salz davon hergestellt werden, in welcher bedeuten:
R(1) Wasserstoff;
Alkyl mit 1, 2, 3, 4, 5, 6, 7 oder 8 C-Atomen, das unsubstituiert ist oder substituiert ist mit 1 oder 2 gleichen oder verschiedenen Resten aus der Reihe F, Cl, CF₃, Methyl, Methoxy, NO₂ oder NR(6)R(7);
- CₙH₂ₙ-Cycloalkyl mit 1, 2, 3, 4, 5, 6 oder 7-C-Atomen;
mit n gleich Null, 1, oder 2;
- CₙH₂ₙ -Phenyl, das unsubstituiert ist oder substituiert ist mit 1 oder 2 gleichen oder verschiedenen Resten aus der Reihe F, Cl, CF₃, NO₂, Methyl, Methoxy, Hydroxy oder NR(6)R(7);
mit n gleich Null, 1 oder 2;
- CₙH₂ₙ-Heteroaryl mit 1, 2, 3, 4, 5, 6, 7, 8 oder 9 C-Atomen, das unsubstituiert ist oder substituiert ist mit einem Rest aus der Reihe F, Cl, CF₃, NO₂, Methyl, Methoxy, Hydroxy oder NR(6)R(7);
mit n gleich Null, 1 oder 2;
R(2) und R(3) unabhängig voneinander
-SR(4) oder -COOR(5);
R(4) und R(5) unabhängig voneinander
Alkyl mit 1, 2, 3, 4, 5, 6, 7 oder 8 C-Atomen, das unsubstituiert ist oder substituiert ist mit 1 oder 2 gleichen oder verschiedenen Resten aus der Reihe F, Cl, CF₃, Methyl, NO₂, Methoxy oder NR(6)R(7);
- CₙH₂ₙ-Cycloalkyl mit 3, 4, 5, 6 oder 7 C-Atomen;
mit n gleich Null, 1 oder 2;
- CₙH₂ₙ-Phenyl, das unsubstituiert ist oder substituiert ist mit 1 oder 2 gleichen oder verschiedenen Resten aus der Reihe F, Cl, CF₃, CN, NO₂, Methyl, Methoxy, Hydroxy oder NR(6)R(7);
mit n gleich Null, 1 oder 2;
- CₙH₂ₙ-Heteroaryl mit 1, 2, 3, 4, 5, 6, 7, 8 oder 9 C-Atomen, das unsubstituiert ist oder substituiert ist mit einem Rest aus der Reihe F, Cl, CF₃, NO₂, Methyl, Methoxy, Hydroxy oder NR(6)R(7);
mit n gleich Null, 1 oder 2;
R(6) und R(7) Methyl.

3. Verfahren gemäß Anspruch 1 und/oder 2, **dadurch gekennzeichnet, daß** Verbindungen der Formel I oder ein Salz davon hergestellt werden, in welcher bedeuten:
R(1) Wasserstoff;
Alkyl mit 1, 2, 3, 4, 5, 6, 7 oder 8 C-Atomen, das unsubstituiert ist oder substituiert ist mit 1 einem Rest aus der Reihe F, Cl, CF₃, Methyl, Methoxy, NO₂ oder NR(6)R(7);
Cycloalkyl mit 1, 2, 3, 4, 5, 6 oder 7-C-Atomen;
Phenyl, das unsubstituiert ist oder substituiert ist mit einem Rest aus der Reihe F, Cl, CF₃, NO₂, Methyl, Methoxy, Hydroxy oder NR(6)R(7);
Heteroaryl mit 1, 2, 3, 4, 5, 6, 7, 8 oder 9 C-Atomen, das unsubstituiert ist oder substituiert ist mit einem Rest aus der Reihe F, Cl, CF₃, NO₂, Methyl, Methoxy, Hydroxy oder NR(6)R(7);
R(2) und R(3) unabhängig voneinander
- SR(4) oder -COOR(5);
R(4) und R(5) unabhängig voneinander
Alkyl mit 1, 2, 3, 4, 5, 6, 7 oder 8 C-Atomen, das unsubstituiert ist oder substituiert ist mit einem Rest aus der Reihe F, Cl, CF₃, Methyl, NO₂, Methoxy oder NR(6)R(7);
Cycloalkyl mit 3, 4, 5, 6 oder 7 C-Atomen;
Phenyl, das unsubstituiert ist oder substituiert ist mit einem Rest aus der Reihe F, Cl, CF₃, CN, NO₂, Methyl, Methoxy, Hydroxy oder NR(6)R(7);
Heteroaryl mit 1, 2, 3, 4, 5, 6, 7, 8 oder 9 C-Atomen, das unsubstituiert ist oder substituiert ist mit einem Rest aus der Reihe F, Cl, CF₃, NO₂, Methyl, Methoxy, Hydroxy oder NR(6)R(7);
R(6) und R(7) Methyl.

4. Verfahren gemäß einem oder mehreren der Ansprüche 1 bis 3, **dadurch gekennzeichnet, daß** Verbindungen der Formel I oder ein Salz davon hergestellt werden, in welcher bedeuten:
R(1) Wasserstoff;
Alkyl mit 1, 2, 3, 4, 5 oder 6 C-Atomen, das unsubstituiert ist oder substituiert ist mit 1 einem Rest aus der Reihe F, Cl, CF₃, Methyl oder Methoxy;
Cycloalkyl mit 1, 2, 3, 4, 5, 6 oder 7-C-Atomen;
Phenyl, das unsubstituiert ist oder substituiert ist mit einem Rest aus der Reihe F, Cl, CF₃, NO₂, Methyl oder Methoxy;
Heteroaryl mit 1, 2, 3, 4, 5, 6, 7, 8 oder 9 C-Atomen, das unsubstituiert ist oder substituiert ist mit einem Rest aus der Reihe F, Cl, CF₃, Methyl oder Methoxy;
R(2) und R(3) unabhängig voneinander
- SR(4) oder -COOR(5);
R(4) und R(5) unabhängig voneinander
Alkyl mit 1, 2, 3, 4, 5 oder 6 C-Atomen, das unsubstituiert ist oder substituiert ist mit einem Rest aus der Reihe F, Cl, CF₃, Methyl oder Methoxy;
Cycloalkyl mit 3, 4, 5, 6 oder 7 C-Atomen;
Phenyl, das unsubstituiert ist oder substituiert ist mit einem Rest aus der Reihe F, Cl, CF₃, CN, Methyl oder Methoxy;
Heteroaryl mit 1, 2, 3, 4, 5, 6, 7, 8 oder 9 C-Atomen, das unsubstituiert ist oder substituiert ist mit einem Rest aus der Reihe F, Cl, CF₃, Methyl oder Methoxy.

5. Verfahren gemäß einem oder mehreren der Ansprüche 1 bis 4, **dadurch gekennzeichnet, daß** Verbindungen der Formel I oder ein Salz davon hergestellt werden, in welcher R(1) Alkyl mit 1, 2, 3, 4, 5 oder 6 C-Atomen bedeutet.

6. Verfahren gemäß einem oder mehreren der Ansprüche 1 bis 5, **dadurch gekennzeichnet, daß** Verbindungen der Formel I oder ein Salz davon hergestellt werden, in welcher
R(1) Alkyl mit 1, 2, 3, 4, 5 oder 6 C-Atomen;
R(2) und R(3) unabhängig voneinander
- SR(4) oder -COOR(5);
R(4) Alkyl mit 1, 2, 3, 4, 5 oder 6 C-Atomen oder -CₙH₂ₙ-Phenyl mit n gleich Null, 1 oder 2 und
R(5) Alkyl mit 1, 2, 3, 4, 5 oder 6 C-Atomen bedeutet.

7. Verfahren gemäß einem oder mehreren der Ansprüche 1 bis 6, **dadurch gekennzeichnet, daß** Verbindungen der Formel I oder ein Salz davon hergestellt werden, in welcher R(2) -S(R4) und R(3) -COOR(5) bedeutet.

8. Verfahren gemäß einem oder mehreren der Ansprüche 1 bis 7, **dadurch gekennzeichnet, daß** die verwendete n-Propylphosphonsäureanhydrid-Menge 0.1-5.0 Mol n-Propylphosphonsäureanhydrid pro Mol Verbindungen der Formel (II) beträgt.

9. Verfahren gemäß Anspruch 8, **dadurch gekennzeichnet, daß** die verwendete n-Propylphosphonsäureanhydrid-Menge 0.2-1.0 Mol n-Propylphosphonsäureanhydrid pro Mol Verbindungen der Formel (II) beträgt.

10. Verfahren gemäß einem oder mehreren der Ansprüche 1 bis 9, **dadurch gekennzeichnet, daß** die Zyklisierung bei einer Temperaturen von 10-130 °C durchgeführt wird.

11. Verfahren gemäß Anspruch 10, **dadurch gekennzeichnet, daß** die Zyklisierung bei einer Temperaturen von 20-80 °C durchgeführt wird.

12. Verfahren gemäß einem oder mehreren der Ansprüche 1 bis 11, **dadurch gekennzeichnet, daß** die Reaktionsdauer 0.25 h bis 3 Tage beträgt.

13. Verfahren gemäß Anspruch 12, **dadurch gekennzeichnet, daß** die Reaktionsdauer 1 bis 48 h beträgt.

## Claims

1. A process for the preparation of compounds of the formula I, 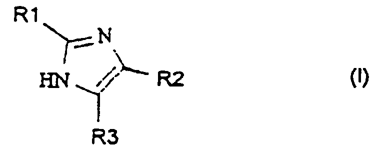 in which
R(1) is hydrogen;
alkyl having 1, 2, 3, 4, 5, 6, 7 or 8 carbon atoms, which is unsubstituted or is substituted by 1, 2 or 3 identical or different radicals from the group consisting of F, Cl, Br, CF₃, methyl, methoxy, NO₂ or NR(6)R(7);
- CₙH₂ₙ-cycloalkyl having 1, 2, 3, 4, 5, 6 or 7 carbon atoms;
where n is equal to 0, 1, 2 or 3;
- CₙH₂ₙ-phenyl, which is unsubstituted or is substituted by 1, 2 or 3 identical or different radicals from the group consisting of F, Cl, Br, CF₃, NO₂, methyl, methoxy, hydroxyl or NR (6) R (7);
where n is equal to 0, 1, 2 or 3;
- CₙH₂ₙ-heteroaryl having 1, 2, 3, 4, 5, 6, 7, 8 or 9 carbon atoms, which is unsubstituted or is substituted by 1, 2 or 3 identical or different radicals from the group consisting of F, Cl, Br, CF₃, NO₂, methyl, methoxy, hydroxyl or NR(6)R(7);
where n is equal to 0, 1, 2 or 3;
R(2) and R(3) independently of one another are
- SR(4) or -COOR(5);
R(4) and R(5) independently of one another are
alkyl having 1, 2, 3, 4, 5, 6, 7 or 8 carbon atoms, which is unsubstituted or is substituted by 1, 2 or 3 identical or different radicals from the group consisting of F, Cl, Br, CF₃, methyl, NO₂, methoxy or NR(6)R(7);
- CₙH₂ₙ-cycloalkyl having 3, 4, 5, 6 or 7 carbon atoms;
where n is equal to 0, 1, 2 or 3;
-CₙH₂ₙ-phenyl, which is unsubstituted or is substituted by 1, 2 or 3 identical or different radicals from the group consisting of F, Cl, Br, CF₃, CN, NO₂, methyl, methoxy, hydroxyl or NR(6)R(7);
where n is equal to 0, 1, 2 or 3;
- CₙH₂ₙ-heteroaryl having 1, 2, 3, 4, 5, 6, 7, 8 or 9 carbon atoms, which is unsubstituted or is substituted by 1, 2 or 3 identical or different radicals from the group consisting of F, Cl, Br, CF₃, NO₂, methyl, methoxy, hydroxyl or NR(6)R(7);
where n is equal to 0, 1, 2 or 3;
R(6) and R(7) independently of one another are
alkyl having 1, 2, 3 or 4 carbon atoms;
or a salt thereof,
which comprises cyclizing compounds of the formula II 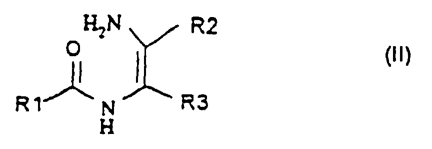 in which R(1), R(2) and R(3) have the meaning defined above, in the presence of n-propylphosphonic anhydride to give compounds of the formula (I), then purifying these by salt formation and, if appropriate, subsequent recrystallization and optionally removing radicals introduced for the protection of other functional groups.

2. The process as claimed in claim 1, wherein compounds of the formula I or a salt thereof are prepared in which:
R(1) is hydrogen;
alkyl having 1, 2, 3, 4, 5, 6, 7 or 8 carbon atoms, which is unsubstituted or is substituted by 1 or 2 identical or different radicals from the group consisting of F, Cl, CF₃, methyl, methoxy, NO₂ or NR(6)R(7);
-CₙH₂ₙ-cycloalkyl having 1, 2, 3, 4, 5, 6 or 7 carbon atoms;
where n is equal to 0, 1 or 2;
-CₙH₂ₙ-phenyl, which is unsubstituted or is substituted by 1 or 2 identical or different radicals from the group consisting of F, Cl, CF₃, NO₂, methyl, methoxy, hydroxyl or NR(6)R(7) ;
where n is equal to 0, 1 or 2;
-CₙH₂ₙ-heteroaryl having 1, 2, 3, 4, 5, 6, 7, 8 or 9 carbon atoms, which is unsubstituted or is substituted by a radical from the group consisting of F, Cl, CF₃, NO₂, methyl, methoxy, hydroxyl or NR(6)R(7);
where n is equal to 0, 1 or 2;
R(2) and R(3) independently of one another are
- SR (4) or -COOR (5);
R(4) and R(5) independently of one another are
alkyl having 1, 2, 3, 4, 5, 6, 7 or 8 carbon atoms, which is unsubstituted or is substituted by 1 or 2 identical or different radicals from the group consisting of F, Cl, CF₃, methyl, NO₂, methoxy or NR(6)R(7); - CₙH₂ₙ-cycloalkyl having 3, 4, 5, 6 or 7 carbon atoms;
where n is equal to 0, 1 or 2;
-CₙH₂ₙ-phenyl, which is unsubstituted or is substituted by 1 or 2 identical or different radicals from the group consisting of F, Cl, CF₃, CN, NO₂, methyl, methoxy, hydroxyl or NR(6)R(7);
where n is equal to 0, 1 or 2;
- CₙH₂ₙ-heteroaryl having 1, 2, 3, 4, 5, 6, 7, 8 or 9 carbon atoms, which is unsubstituted or is substituted by a radical from the group consisting of F, Cl, CF₃, NO₂, methyl, methoxy, hydroxyl or NR(6)R(7);
where n is equal to 0, 1 or 2;
R(6) and R(7) are
methyl.

3. The process as claimed in claim 1 and/or 2, wherein compounds of the formula I or a salt thereof are prepared in which:
R(1) is hydrogen;
alkyl having 1, 2, 3, 4, 5, 6, 7 or 8 carbon atoms, which is unsubstituted or is substituted by a radical from the group consisting of F, Cl, CF₃, methyl, methoxy, NO₂ or NR(6)R(7);
cycloalkyl having 1, 2, 3, 4, 5, 6 or 7 carbon atoms;
phenyl, which is unsubstituted or is substituted by a radical from the group consisting of F, Cl, CF₃, NO₂, methyl, methoxy, hydroxyl or NR(6)R(7);
heteroaryl having 1, 2, 3, 4, 5, 6, 7, 8 or 9 carbon atoms, which is unsubstituted or is substituted by a radical from the group consisting of F, Cl, CF₃, NO₂, methyl, methoxy, hydroxyl or NR (6) R (7);
R(2) and R(3) independently of one another are
- SR(4) or -COOR (5);
R(4) and R(5) independently of one another are
alkyl having 1, 2, 3, 4, 5, 6, 7 or 8 carbon atoms, which is unsubstituted or is substituted by a radical from the group consisting of F, Cl, CF₃, methyl, NO₂, methoxy or NR(6)R(7);
cycloalkyl having 3, 4, 5, 6 or 7 carbon atoms;
phenyl, which is unsubstituted or is substituted by a radical from the group consisting of F, Cl, CF₃, CN, NO₂, methyl, methoxy, hydroxyl or NR(6)R(7);
heteroaryl having 1, 2, 3, 4, 5, 6, 7, 8 or 9 carbon atoms, which is unsubstituted or is substituted by a radical from the group consisting of F, Cl, CF₃, NO₂, methyl, methoxy, hydroxyl or NR(6)R(7);
R(6) and R(7) are
methyl.

4. The process as claimed in one or more of claims 1 to 3, wherein compounds of the formula 1 or a salt thereof are prepared in which:
R(1) is hydrogen;
alkyl having 1, 2, 3, 4, 5 or 6 carbon atoms, which is unsubstituted or is substituted by a radical from the group consisting of F, Cl, CF₃, methyl or methoxy;
cycloalkyl having 1, 2, 3, 4, 5, 6 or 7 carbon atoms;
phenyl, which is unsubstituted or is substituted by a radical from the group consisting of F, Cl, CF₃, NO₂, methyl or methoxy;
heteroaryl having 1, 2, 3, 4, 5, 6, 7, 8 or 9 carbon atoms, which is unsubstituted or is substituted by a radical from the group consisting of F, Cl, CF₃, methyl or methoxy;
R(2) and R(3) independently of one another are
- SR(4) or -COOR(5);
R(4) and R(5) independently of one another are
alkyl having 1, 2, 3, 4, 5 or 6 carbon atoms, which is unsubstituted or is substituted by a radical from the group consisting of F, Cl, CF₃, methyl or methoxy;
cycloalkyl having 3, 4, 5, 6 or 7 carbon atoms;
phenyl, which is unsubstituted or is substituted by a radical from the group consisting of F, Cl, CF₃, CN, methyl or methoxy;
heteroaryl having 1, 2, 3, 4, 5, 6, 7, 8 or 9 carbon atoms, which is unsubstituted or is substituted by a radical from the group consisting of F, Cl, CF₃, methyl or methoxy.

5. The process as claimed in one or more of claims 1 to 4, wherein compounds of the formula I or a salt thereof are prepared in which R(1) is alkyl having 1, 2, 3, 4, 5 or 6 carbon atoms.

6. The process as claimed in one or more of claims 1 to 5, wherein compounds of the formula I or a salt thereof are prepared in which
R(1) is alkyl having 1, 2, 3, 4, 5 or 6 carbon atoms;
R(2) and R(3) independently of one another are
- SR(4) or -COOR(5);
R(4) is alkyl having 1, 2, 3, 4, 5 or 6 carbon atoms or
- CₙH₂ₙ-phenyl where n is equal to 0, 1 or 2 and
R(5) is alkyl having 1, 2, 3, 4, 5 or 6 carbon atoms.

7. The process as claimed in one or more of claims 1 to 6, wherein compounds of the formula I or a salt thereof are prepared in which R(2) is -SR(4) and R(3) is - COOR(5).

8. The process as claimed in one or more of claims 1 to 7, wherein the amount of n-propylphosphonic anhydride used is 0.1-5.0 mol of n-propylphosphonic anhydride per mole of compounds of the formula (II).

9. The process as claimed in claim 8, wherein the amount of n-propylphosphonic anhydride used is 0.2-1.0 mol of n-propylphosphonic anhydride per mole of compounds of the formula (II).

10. The process as claimed in one or more of claims 1 to 9, wherein the cyclization is carried out at temperatures of 10-130°C.

11. The process as claimed in claim 10, wherein the cyclization is carried out at temperatures of 20-80°C.

12. The process as claimed in one or more of claims 1 to 11, wherein the reaction time is 0.25.h to 3 days.

13. The process as claimed in claim 12, wherein the reaction time is 1 to 48 h.

## Revendications

1. Procédé pour la préparation de composés de formulé générale I 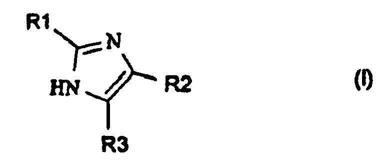 dans laquelle
R(1) signifie
hydrogène ;
alkyle comprenant 1, 2, 3, 4, 5, 6, 7 ou 8 atomes de carbone, qui est non substitué ou substitué par 1, 2 ou 3 radicaux identiques ou différents de la série F, Cl, Br, CF₃, méthyle, méthoxy, NO₂ ou NR(6)R(7) ;
- CₙH₂ₙ-cycloalkyle avec 1, 2, 3, 4, 5, 6 ou 7 atomes de carbone ;
avec n valant 0, 1, 2 ou 3 ;
- CₙH₂ₙ-phényle, qui est non substitué ou substitué par 1, 2 ou 3 radicaux identiques ou différents de la série F, Cl, Br, CF₃, NO₂, méthyle, méthoxy, hydroxy ou NR(6)R(7) ;
avec n valant zéro, 1, 2 ou 3 ;
- CₙH₂ₙ-hétéroaryle avec 1, 2, 3, 4, 5, 6, 7, 8 ou 9 atomes de carbone, qui est non substitué ou substitué par 1, 2 ou 3 radicaux identiques ou différents de la série F, Cl, Br, CF₃, NO₂, méthyle, méthoxy, hydroxy ou NR(6)R(7) ;
avec n valant 0, 1, 2 ou 3 ;
R(2) et R(3) signifient indépendamment l'un de l'autre
- SR(4) ou -COOR(5) ;
R(4) et R(5) signifient indépendamment l'un de l'autre
alkyle avec 1, 2, 3, 4, 5, 6, 7 ou 8 atomes de carbone, qui est non substitué ou substitué par 1, 2 ou 3 radicaux identiques ou différents de la série F, Cl, Br, CF₃, méthyle, NO₂, méthoxy ou NR(6)R(7) ;
- CₙH₂ₙ-cycloalkyle avec 3, 4, 5, 6 ou 7 atomes de carbone ;
avec n valant 0, 1, 2 ou 3 ;
- CₙH₂ₙ-phényle, qui est non substitué ou substitué par 1, 2 ou 3 radicaux identiques ou différents de la série F, Cl, Br, CF₃, CN, NO₂, méthyle, méthoxy, hydroxy ou NR(6)R(7) ;
avec n valant 0, 1, 2 ou 3 ;
- CₙH₂ₙ-hétéroaryle avec 1, 2, 3, 4, 5, 6, 7, 8 ou 9 atomes de carbone, qui est substitué ou non substitué par 1, 2 ou 3 radicaux identiques ou différents de la série F, Cl, Br, CF₃, NO₂, méthyle, méthoxy, hydroxy ou NR(6)R(7) ;
avec n valant 0, 1, 2 ou 3 ;
R(6) et R(7) signifient indépendamment l'un de l'autre
alkyle avec 1, 2, 3 ou 4 atomes de carbone ;
ou un sel de ceux-ci,
**caractérisé en ce qu'**on cyclise des composés de formule II 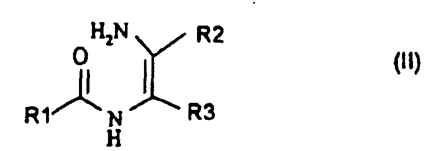 dans laquelle R(1), R(2) et R(3) ont la signification définie ci-dessus en présence d'anhydride de l'acide n-propylphosphonique en composés de formule (I), on les purifie ensuite par formation de sel et, le cas échéant, recristallisation consécutive et on dissocie les radicaux le cas échéant introduits pour la protection d'autres groupes fonctionnels.

2. Procédé selon la revendication 1, **caractérisé en ce qu'**on prépare des composés de formule I ou un sel de ceux-ci, dans laquelle
R(1) signifie
hydrogène ;
alkyle avec 1, 2, 3, 4, 5, 6, 7 ou 8 atomes de carbone, qui est non substitué ou substitué par 1 ou 2 radicaux identiques ou différents de la série F, Cl, CF₃, méthyle, méthoxy, NO₂ ou NR(6)R(7) ;
- CₙH₂ₙ-cycloalkyle avec 1, 2, 3, 4, 5, 6 ou 7 atomes de carbone ;
avec n valant 0, 1, ou 2 ;
- CₙH₂ₙ-phényle qui est substitué ou non substitué par 1 ou 2 radicaux identiques ou différents de la série F, Cl, CF_{3,} NO₂, méthyle, méthoxy, hydroxy ou NR(6)R(7) ;
avec n valant 0, 1 ou 2 ;
- CₙH₂ₙ-hétéroaryle avec 1, 2, 3, 4, 5, 6, 7, 8 ou 9 atomes de carbone, qui est non substitué ou substitué par un radical de la série F, Cl, CF₃, NO₂, méthyle, méthoxy, hydroxy ou NR(6)R(7) ;
avec n valant 0, 1 ou 2 ;
R(2) et R(3) signifient indépendamment l'un de l'autre
- SR(4) ou -COOR(5) ;
R(4) et R(5) signifient indépendamment l'un de l'autre
alkyle avec 1, 2, 3, 4, 5, 6, 7 ou 8 atomes de carbone, qui est non substitué ou substitué par 1 ou 2 radicaux identiques ou différents de la série F, Cl, CF₃, méthyle, NO₂, méthoxy ou NR(6)R(7) ;
- CₙH₂ₙ-cycloalkyle avec 3, 4, 5, 6 ou 7 atomes de carbone ;
avec n valant 0, 1 ou 2 ;
- CₙH₂ₙ-Phényle, qui est non substitué ou substitué par 1 ou 2 radicaux identiques ou différents de la série F, Cl, CF₃, CN, NO₂, méthyle, méthoxy, hydroxy ou NR(6)R(7) ;
avec n valant 0, 1 ou 2 ;
- CₙH₂ₙ-hétéroaryle avec 1, 2, 3, 4, 5, 6, 7, 8 ou 9 atomes de carbone, qui est non substitué ou substitué par un radical de la série F, Cl, CF₃, NO₂, méthyle, méthoxy, hydroxy ou NR(6)R(7) ;
avec n valant 0, 1 ou 2 ;
R(6) et R(7) signifient méthyle.

3. Procédé selon la revendication 1 et/ou 2, **caractérisé en ce qu'**on prépare des composés de formule I ou un sel de ceux-ci, dans laquelle :
R(1) signifie
hydrogène ;
alkyle avec 1, 2, 3, 4, 5, 6, 7 ou 8 atomes de carbone, qui est non substitué ou substitué par un radical de la série F, Cl, CF₃, méthyle, méthoxy, NO₂ ou NR(6)R(7) ;
cycloalkyle avec 1, 2, 3, 4, 5, 6 ou 7 atomes de carbone ;
phényle, qui est non substitué ou substitué par un radical de la série F, Cl, CF₃, NO₂, méthyle, méthoxy, hydroxy ou NR(6)R(7) ;
hétéroaryle avec 1, 2, 3, 4, 5, 6, 7, 8 ou 9 atomes de carbone, qui est non substitué ou substitué par un radical de la série F, Cl, CF₃, NO₂, méthyle, méthoxy, hydroxy ou NR(6)R(7) ;
R(2) et R(3) signifient indépendamment l'un de l'autre
- SR(4) ou -COOR(5) ;
R(4) et R(5) signifient indépendamment l'un de l'autre
alkyle avec 1, 2, 3, 4, 5, 6, 7 ou 8 atomes de carbone, qui est non substitué ou substitué par un radical de la série F, Cl, CF₃, méthyle, NO₂, méthoxy ou NR(6)R(7) ;
cycloalkyle avec 3, 4, 5, 6 ou 7 atomes de carbone ;
phényle, qui est non substitué ou substitué par un radical de la série F, Cl, CF₃, CN, NO₂, méthyle, méthoxy, hydroxy ou NR(6)R(7) ;
hétéroaryle avec 1, 2, 3, 4, 5, 6, 7, 8 ou 9 atomes de carbone, qui est non substitué ou substitué par un radical de la série F, Cl, CF₃, NO₂, méthyle, méthoxy, hydroxy ou NR(6)R(7) ;
R(6) et R(7) signifient méthyle.

4. Procédé selon l'une ou plusieurs des revendications 1 à 3, **caractérisé en ce qu'**on prépare des composés de formule I ou un sel de ceux-ci dans laquelle :
R(1) signifie
hydrogène ;
alkyle avec 1, 2, 3, 4, 5 ou 6 atomes de carbone, qui est non substitué ou substitué par un radical de la série F, Cl, CF₃, méthyle ou méthoxy ;
cycloalkyle avec 1, 2, 3, 4, 5, 6 ou 7 atomes de carbone ;
phényle, qui est non substitué ou substitué par un radical de la série F, Cl, CF₃, NO₂, méthyle ou méthoxy ;
hétéroaryle avec 1, 2, 3, 4, 5, 6, 7, 8 ou 9 atomes de carbone, qui est non substitué ou substitué par un radical de la série F, Cl, CF₃, méthyle ou méthoxy ;
R(2) et R(3) signifient indépendamment l'un de l'autre
-SR(4) ou -COOR(5) ;
R(4) et R(5) signifient indépendamment l'un de l'autre
alkyle avec 1, 2, 3, 4, 5 ou 6 atomes de carbone, qui est non substitué ou substitué par un radical de la série F, Cl, CF₃, méthyle ou méthoxy ;
cycloalkyle avec 3, 4, 5, 6 ou 7 atomes de carbone ;
phényle, qui est non substitué ou substitué par un radical de la série F, Cl, CF₃, CN, méthyle ou méthoxy ;
hétéroaryle avec 1, 2, 3, 4, 5, 6, 7, 8 ou 9 atomes de carbone, qui est non substitué ou substitué par un radical de la série F, Cl, CF₃, méthyle ou méthoxy.

5. Procédé selon l'une ou plusieurs des revendications des revendications 1 à 4, **caractérisé en ce qu'**on prépare des composés de formule 1 ou un sel de ceux-ci dans laquelle R(1) signifie alkyle avec 1, 2, 3, 4, 5 ou 6 atomes de carbone.

6. Procédé selon l'une ou plusieurs des revendications 1 à 5, **caractérisé en ce qu'**on prépare des composés de formule I ou un sel de ceux-ci, dans laquelle
R(1) signifie
alkyle avec 1, 2, 3, 4, 5 ou 6 atomes de carbone ;
R(2) et R(3) signifient indépendamment l'un de l'autre
- SR(4) ou -COOR(5) ;
R(4) signifie
alkyle avec 1, 2, 3, 4, 5 ou 6 atomes de carbone
ou -CₙH₂ₙ-phényle avec n valant 0, 1 ou 2 et
R(5) signifie
alkyle avec 1, 2, 3, 4, 5 ou 6 atomes de carbone.

7. Procédé selon l'une ou plusieurs des revendications 1 à 6, **caractérisé en ce qu'**on prépare des composés de formule I ou un sel de ceux-ci, dans laquelle R(2) signifie -S(R4) et R(3) signifie -COOR(5).

8. Procédé selon l'une ou plusieurs des revendications 1 à 7, **caractérisé en ce que** la quantité utilisée d'anhydride de l'acide n-propylphosphonique est de 0,1 à 5,0 moles d'anhydride de l'acide n-propylphosphonique par mole de composés de formule (II).

9. Procédé selon la revendication 8, **caractérisé en ce que** la quantité utilisée d'anhydride de l'acide n-propylphosphonique est de 0,2 à 1,0 mole d'anhydride de l'acide n-propylphosphonique par mole de composés de formule (II).

10. Procédé selon l'une ou plusieurs des revendications 1 à 9, **caractérisé en ce que** la cyclisation est réalisée à une température de 10 à 130°C.

11. Procédé selon la revendication 10, **caractérisé en ce que** la cyclisation est réalisée à une température de 20 à 80°C.

12. Procédé selon l'une ou plusieurs des revendications 1 à 11, **caractérisé en ce que** la durée de réaction est de 0,25 h à 3 jours.

13. Procédé selon la revendication 12, **caractérisé en ce que** la durée de réaction est de 1 à 48 heures.
